# EUROPÄISCHE PATENTSCHRIFT

(11) **EP 1 172 082 B1**
(45) Veröffentlichungstag und Bekanntmachung des Hinweises auf die Patenterteilung: **31.08.2005**
(21) Anmeldenummer: 01114734.5
(22) Anmeldetag: 22.06.2001
(51) Int. Cl.: A61K 7/13

(54) **Mittel zum Färben von menschlichen Haaren**
Process for dyeing human hair
Procédé pour la teinture des cheveux humains

(30) Priorität: 23.06.2000 DE 10031016
(43) Veröffentlichungstag der Anmeldung: 16.01.2002
(60) Teilanmeldung: 03017991.5
(73) Patentinhaber: KPSS Kao Professional Salon Services GmbH, 64280 Darmstadt (DE)
(72) Erfinder: Grit, Mustafa, Dr., 64579 Gernsheim (DE)

(56) Entgegenhaltungen:
- EP-A- 0 819 422
- EP-A- 0 970 684
- WO-A-95/01772
- NUNEZ-TOLIN V. ET AL: 'Surface Tension Measurements by the Drop-Weight Method for Continuously Varying Surfactant Concentration' JOURNAL OF COLLOID AND INTERFACE SCIENCE Bd. 85, Nr. 2, 1982, Seiten 597 - 600

## Beschreibung

Die vorliegende Erfindung betrifft ein Mittel zum Färben von menschlichen Haaren, das eine Haarfärbung mit erhöhter Färbeintensität bewirkt.

Es ist allgemein bekannt, daß Haarfärbemittel in zwei Kategorien aufgeteilt werden, nämlich einerseits die permanenten Haarfärbemittel, die grundsätzlich Haarfarbstoffvorprodukte enthalten, die zusammen mit Oxidationsmitteln je nach Zusammensetzung die gewünschte Färbung auf dem Haar entwickeln; und andererseits semipermanente Haarfärbemittel, die direktziehende Farbstoffe enthalten, die zur Entwicklung ihrer Färbeleistung keinerlei Oxidationsmittelzusatzes bedürfen. Dementsprechend sind die Färbungen auch weniger dauerhaft als diejenigen mit Permanentfarbstoffen erzielbaren.

Diese Farbzusammensetzungen auf Basis direktziehender Farbstoffe werden in der Regel entweder als Tönungsshampoos, als Farblotionen oder als Tönungsfestiger, gegebenenfalls auch als Aerosolschaum, appliziert.

Die verwendeten direktziehenden Farbstoffe sind in der Regel kationischer Natur; als weiteren wesentlichen Bestandteil enthalten die als Spülung verwendeten Zusammensetzungen auch kationische Tenside, insbesondere quaternäre Ammoniumsalze.

Die mit diesen Zusammensetzungen erzielbare Farbintensität und Dauerhaftigkeit ist jedoch nicht immer befriedigend.

Es wurde nunmehr gefunden, daß sich Haarfärbungen mit verbesserter Farbintensität und -stabilität sowie großer Gleichmäßigkeit dann erzielen lassen, wenn die Färbung mit einem Haarfärbemittel erfolgt, das
a) mindestens einen direktziehenden kationischen Haarfarbstoff, der allgemeinen Formel 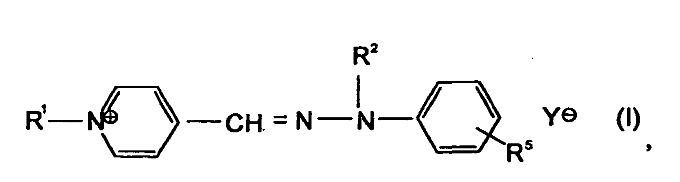 worin R¹, und R², für Wasserstoff, eine CH₃- oder C₂H₅-Gruppe stehen und R⁵ Wasserstoff, -OCH₃ oder -OC₂H₅ bedeutet,
   und Y⁻ in allen Fällen ein Anion bedeuten
   und
b) mindestens ein amphoteres und/oder zwitterionisches Tensid enthält.

Bevorzugt eingesetzte Farbstoffe nach den obengenannten Formeln sind solche entsprechend (I), worin R¹ und R² eine Methylgruppe und R⁵ Wasserstoff oder eine Methoxygruppe bedeuten.

Das Anion Y⁻ ist vorzugsweise C₂H₅SO₄⁻, CH₃SO₄⁻ oder Cl⁻.

Der Anteil dieser direktziehenden kationischen Farbstoff in den erfindungsgemäßen Mitteln beträgt vorzugsweise etwa 0,001 bis etwa 5, insbesondere etwa 0,05 bis 1,5, vor allem etwa 0,1 bis etwa 1 Gew.-%, berechnet auf das Haarfärbemittel.

Neben den essentiellen Farbstoffen der allgemeinen Formeln (I) können auch noch weitere, bekannte synthetische und natürliche direktziehende Haarfarbstoffe eingesetzt werden.

Als solche Haarfarbstoffe können im Prinzip alle für diesen Zweck vorgeschlagenen kationischen Farbstoffe verwendet werden.

Bevorzugt sind die sogenannten "Arianor"-Farbstoffe; vgl. K. Schrader, "Grundlagen und Rezepturen der Kosmetika", 2. Aufl. (1989), S. 811.

Besonders geeignete basische (kationische) Farbstoffe sind:
- Basic Blue 6,: C.I.-No. 51,175;
- Basic Blue 7,: C.I.-No. 42,595;
- Basic Blue 9,: C.I.-No. 52,015;
- Basic Blue 26,: C.I.-No. 44,045;
- Basic Blue 41,: C.I.-No. 11,154;
- Basic Blue 99,: C.I.-No. 56,059;
- Basic Brown 4,: C.I.-No. 21,010;
- Basic Brown 16,: C.I.-No. 12,250;
- Basic Brown 17,: C.I.-No. 12,251;
- Natural Brown 7,: C.I.-No. 75,500;
- Basic Green 1,: C.I.-No. 42,040;
- Basic Red 2,: C.I.-No. 50,240;
- Basic Red 22,: C.I.-No. 11,055;
- Basic Red 76,: C.I.-No. 12,245;
- Basic Violet 1,: C.I.-No. 42,535;
- Basic Violet 3,: C.I.-No. 42,555;
- Basic Violet 10,: C.I.-No. 45,170;
- Basic Violet 14,: C.I.-No. 42,510;
- Basic Yellow 57,: C.I.-No. 12,719.

Im Rahmen der Erfindung geeignete amphotere bzw. zwitterionische Tenside sind insbesondere Alkylamidobetaine der allgemeinen Formel 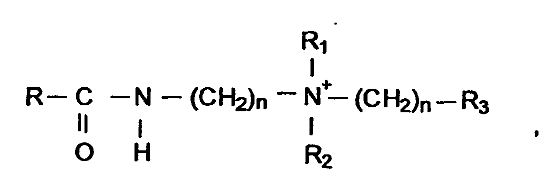 wobei R eine C₈-C₁₈-Alkylgruppe, z. B. einen Cocoalkylrest; R₁ und R₂ einen niederen C₁-C₄-Alkyl- oder -Hydroxyalkylrest, insbesondere eine Methyl-, Ethyl- und/oder Hydroxyethylgruppe; R₃ eine COO- oder -SO₃⁻-Gruppe; und n 1 bis 3 bedeuten.

Auch Betaine der allgemeinen Formel 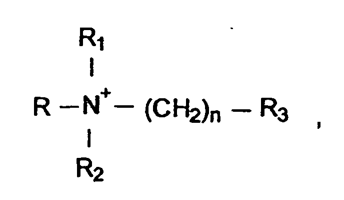 wobei R, R₁, R₂, R₃ und n die obengenannte Bedeutung haben, sind bevorzugt.

Solche Betaine sind beispielsweise mit der folgenden Formel 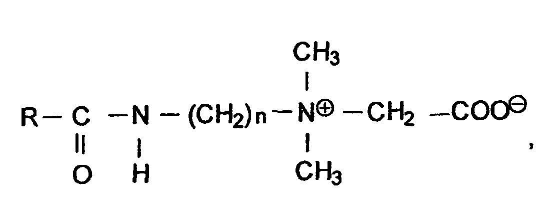 und/oder 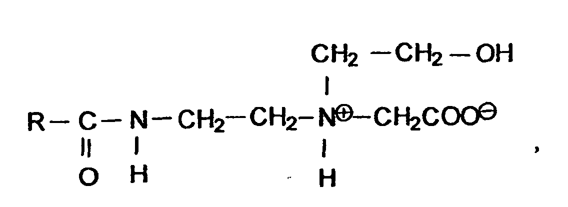 ein Sulfobetain mit der Formel 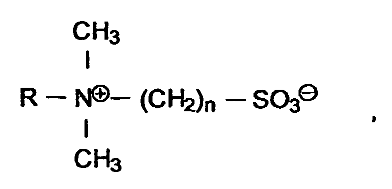 und ein Betain mit der Formel 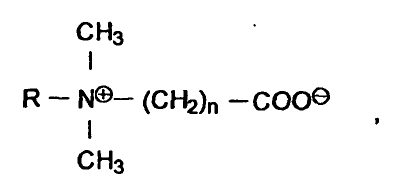 wo R eine C₈-C₁₈-Alkylgruppe und n 1 bis 3 bedeuten, gekennzeichnet.

Geeignet sind insbesondere auch Handelsprodukte wie beispielsweise "Tegobetaine®", "Dehytone®" wie "AB 30", "G" und "K", "Lonzaine®", "Varion®" wie "ADG" und "CAS", "Lexaine®", "Chembetaine®", "Mirataine®", "Rewoteric®", "Schercotaine®", "Monteine LCQ®"; ""Alkateric®", "Amonyl®", "Amphosol®", "Cycloteric BET®", "Emcol®", "Empigen®", "Mackam®", "Monateric®", "Unibetaine®" und "Velvetex®".

Weitere bevorzugte amphotere bzw. zwitterionische Tenside sind ausgewählt aus der Gruppe C₈-C₁₈-Alkylhydroxysulfobetain, ein Carboxymethyl-C₈-C₁₈-Alkylpolypropylamin und/oder Verbindungen der Formel 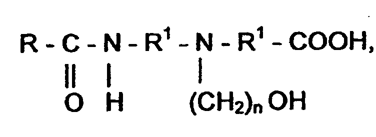 oder 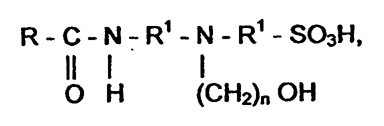 worin R eine C₈-C₂₀-Alk(en)ylgruppe, vorzugsweise eine C₈-C₁₄-Alkylgruppe, R¹ eine gegebenenfalls hydroxysubstituierte, gerad- oder verzweigtkettige Alkylengruppe mit 1 bis 3 Kohlenstoffatomen und n 1 bis 3 bedeuten, bzw. deren wasserlöslichen Salzen.

Bevorzugt sind dabei Natriumcaproamphoacetat, Natriumcaproamphopropionat, Natriumcaproamphohydroxypropylsulfonat, Natriumcocoamphopropionat bzw. - acetat, Natriumcocoamphohydroxypropylsulfonat, Natriumisostearoamphoglycinat und auch Natriumcarboxymethylmethylcocopolypropylamin der Formel 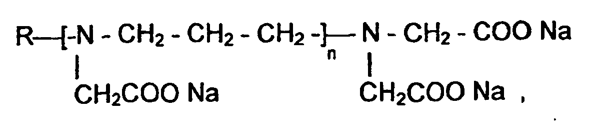 wobei R einen Cocosalkylrest und n vorzugsweise 1 bis 4 bedeuten.

Die zwitterionischen bzw. amphoteren Tenside sind vorzugsweise in einer Menge 0,1 bis 7,5 Gew.-%, insbesondere 0,25 bis 5 Gew.-%, berechnet auf die Gesamtsammensetzung, enthalten.

Ein fakultativer Bestandteil der erfindungsgemäßen Zusammensetzungen ist ein anionischer, vorzugsweise wasserlöslicher UV-Absorber, der vorzugsweise in einer Menge von 0,1 bis 5, insbesondere 0,25 bis 2,5 Gew.-%, berechnet auf die Gesamtzusammensetzung des Mittels, eingesetzt wird.

Besonders geeignete wasserlösliche UV-Absorber mit anionischen Gruppen sind beispielsweise 5-Benzoyl-4-hydroxy-2-methoxybenzolsulfonsäure (Benzophenone-4), dessen Natriumsalz (Benzophenone-5) und 2,2'-Dihydroxy-4,4'-dimethoxy-3,3'-disulfobenzophenon bzw. dessen Dinatriumsalz (Benzophenone-9) sowie Phenylbenzimidazolsulfonsäure (Eusolex® 232); jedoch können auch andere wasserlösliche UV-Absorber eingesetzt werden.

Das bevorzugte Gewichtsverhältnis von zwitterionischem bzw. amphoteren Tensid zu anionischem UV-Absorber soweit vorhanden, liegt vorzugsweise bei etwa 1 : 1 bis etwa 3 : 1.

Die erfindungsgemäßen Zusammensetzungen können noch mindestens ein kationisches Tensid, insbesondere in einer Menge von 0,1 bis 7,5, vorzugsweise 0,25 bis 5, besonders bevorzugt 0,5 bis 2,5 Gew.-%, der Gesamtzusammensetzung, enthalten.

Geeignete langkettige quaternäre Ammoniumverbindungen, die als kationische Tenside allein oder im Gemisch miteinander eingesetzt werden können, sind insbesondere Cetyltrimethylammoniumchlorid, Dimethyldicetylammoniumchlorid, Trimethylcetyl-ammoniumbromid, Stearyltrimethylammoniumchlorid, Dimethylstearylbenzyl-ammoniumchlorid, Benzyltetradecyldimethylammoniumchlorid, Dimethyldihydriertes-Talgammoniumchlorid, Laurylpyridiniumchlorid, Lauryldimethylbenzyl-ammoniumchlorid, Lauryltrimethylammoniumchlorid, Tris(oligooxyethyl)alkylammoniumphosphat, Cetylpyridiniumchlorid, etc.

Gut geeignet sind auch die in der EP-A 472 107 geoffenbarten quaternären Ammoniumsalze.

Im Prinzip sind alle quaternären Ammoniumverbindungen, wie sie im "CTFA International Cosmetic Ingredient Dictionary", Fourth Ed. (1991), unter dem Trivialnamen "Quaternium" aufgeführt sind, geeignet.

Die Zusammensetzung kann natürlich zusätzlich die in solchen Konditionier ungsmitteln üblichen Bestandteile enthalten; es wird, zur Vermeidung von Wiederholungen, wiederum auf K. Schrader, "Grundlagen und Rezepturen der Kosmetika", 2. Aufl. (1989), S. 722- 771, verwiesen.

Auch nichtionische Tenside können, insbesondere im Gemisch mit kationaktiven Tensiden, Verwendung finden, beispielsweise Aminoxide in einer Menge von etwa 0,25 bis etwa 5, vorzugsweise etwa 0,5 bis etwa 3,5 Gew.-%, berechnet auf die Gesamtzusammensetzung des Mittels.

Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl- oder -ethylaminoxide, C₁₂-C₁₈-Alkyldi(hydroxyethyl)- oder (hydroxypropyl)-aminoxide, oder auch Aminoxide mit Ethylenoxid- und /oder Propylenoxidgruppen in der Alkylkette.

Geeignete Tenside sind weiterhin die bekannten C₈-C₁₈-Alkylpolyglucoside, insbesondere mit einem Polykondensationsgrad von 1,2 bis 3.

Die erfindungsgemäßen Haarfärbemittel können die in solchen wäßrigen Zubereitungen üblichen Stoffe enthalten.

Dies sind beispielsweise synthetische oder natürliche haarkonditionierende Polymere, vorzugsweise in einer Menge von 0,1 bis 2,5, insbesondere 0,25 bis 1,5 Gew.-% der Gesamtzusammensetzung.

Als geeignete kationische Polymere sind neben den altbekannten quaternären Cellulosederivaten des Typs "Polymer JR" insbesondere quaternisierte Homo- und Copolymere des Dimethyldiallylammoniumchlorids, wie sie unter dem Handelsnamen "Merquat" im Handel sind, quaternäre Vinylpyrrolidon-Copolymere, insbesondere mit Dialkylaminoalkyl(meth)acrylaten, wie sie unter dem Namen "Gafquat" bekannt sind, Copolymerisate aus Vinylpyrrolidon und Vinylimidazoliniummethochlorid, die unter dem Handelsnamen "Luviquat" angeboten werden, Polyamino-Polyamid-Derivate, beispielsweise Copolymere von Adipinsäure-Dimethylaminohydroxypropyldiethylentriamin, wie sie unter dem Namen "Cartaretine F" vertrieben werden, sowie auch bisquaternäre langkettige Ammoniumverbindungen der in der US-PS 4 157 388 beschriebenen Harnstoff-Struktur, die unter dem Handelsnamen "Mirapol A 15" im Handel sind, bekannt.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Anstelle der kationischen Polymeren oder in Kombination mit denselben können auch nichtionische Polymere verwendet werden. Als geeignete nichtionische Polymere werden vor allem Vinylpyrrolidon-Homo- und Copolymerisate, insbesondere Polyvinylpyrrolidon selbst, Copolymere aus Vinylpyrrolidon und Vinylacetat oder Terpolymerisate aus Vinylpyrrolidon, Vinylacetat und Vinylpropionat, wie sie beispielsweise von der Firma BASF unter dem Handelsnamen "Luviskol" vertrieben werden, eingesetzt.

Es können jedoch auch (Co-)Polymerisate aus den verschiedenen Acryl- und Methacrylestern, Acrylamid und Methacrylamid, beispielsweise Polyacrylamid mit Molgewichten von über 100.000, Dimethylhydantoin-Formaldehyd-Harze, etc., verwendet werden. Selbstverständlich sind auch Mischungen aus verschiedenen nichtionischen Polymeren verwendbar.

Geeignet sind schließlich auch noch amphotere Polymere, z. B. die unter der Bezeichnung "Amphomer" vertriebenen Copolymerisate aus N-Octylacrylamid, N-Butylamino-ethylmethacrylat und Acrylsäure.

Die erfindungsgemäßen Färbemittel können die in solchen Zusammensetzungen üblichen Zusätze enthalten, deren Art und Charakter von der Applikationsform des Mittels abhängig ist. Es sind dies Fette, Fettalkohole, Emulgatoren, pH-Regulatoren, Lösungs- und Verbindungsmittel, Lösungsvermittler, Konservierungsmittel, Parfums, etc.

Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle, Polyethylenglykole, etc.

Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglyceryl-fettsäureester wie PEG-7-glycerylcocoat, Cetylpalmitat, etc.

Diese hydrophoben Komponenten sind in den erfindungsgemäßen Zusammensetzungen vorzugsweise in einer Gesamtmenge von etwa 0,5 bis etwa 10, insbesondere etwa 1 bis 7,5, vor allem etwa 1,5 bis 5 Gew.-%, berechnet auf die Gesamtzusammensetzung, enthalten.

Eine Zusammenfassung der Herstellung solcher Mittel findet sich in der bereits erwähnten Monographie von K. Schrader, S. 798 bis 815, insbesondere S. 804 ff.

Die erfindungsgemäßen Färbemittel liegen als Emulsion, Dispersion oder (gegebenenfalls verdickte, d. h. als Gel) Lösung vor und können auch als Aerosolschaum konfektioniert werden. Diese Zusammensetzungen und ihre Herstellung sind dem Fachmann grundsätzlich bekannt und bedürfen daher keiner näheren Erläuterung.

Der pH-Wert der erfindungsgemäßen Haarfärbemittel liegt vorzugsweise bei etwa 3 bis 8,5, insbesondere zwischen 4 und 6.
Die folgenden Beispiele beschreiben die Zusammensetzung der erfindungsgegemäßen Mittel.
Farbspülungen der folgenden Zusammensetzung wurden durch Vermischen der Bestandteile hergestellt:

### Beispiel 1

### Farbkonditioner

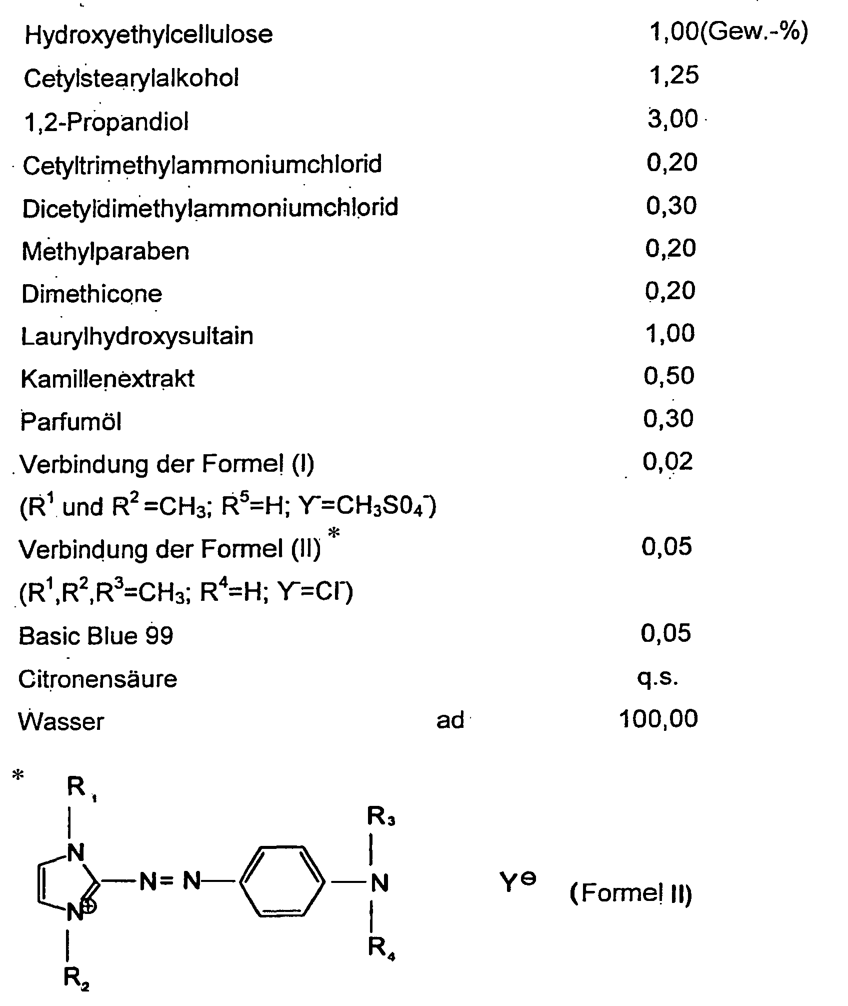

Es wurde eine ausdrucksvolle, glänzende, gleichmäßig intensive Braunfärbung erhalten.

## Patentansprüche

1. Haarfärbemittel, enthaltend
a) mindestens einen Farbstoff, ausgewählt aus einer Verbindung der Gruppe 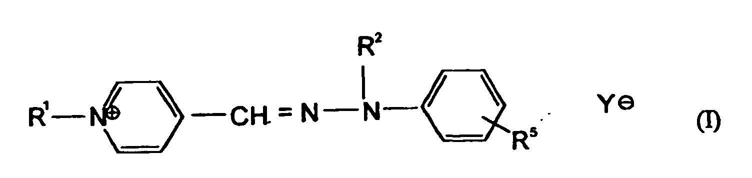 worin R¹, und R², für Wasserstoff, eine CH₃- oder C₂H₅-Gruppe stehen und R⁵ Wasserstoff, -OCH₃ oder -OC₂H₅ bedeutet,
und Y⁻ ein Anion bedeuten,
b) mindestens ein amphoteres und/oder zwitterionisches Tensid, und
c) mindestens ein kationisches Tensid.

2. Haarfärbemittel nach Anspruch 1, enthaltend einen Farbstoff der allgemeinen Formel (I), worin R¹ und R² eine Methylgruppe, R⁵ Wasserstoff und Y⁻ eine Methosulfatgruppe bedeuten.

3. Haarfärbemittel nach Anspruch 1, enthaltend einen Farbstoff der allgemeinen Formel (I), worin R¹ und R² je eine Methylgruppe, R⁵ eine Methoxygruppe und Y Cl⁻ bedeuten.

4. Haarfärbemittel nach einem oder mehreren der Ansprüche 1 bis 3, enthaltend Cocoamidobetain.

## Claims

1. Hair dyeing composition, comprising
a) at least one dyestuff, selected from a compound of the group 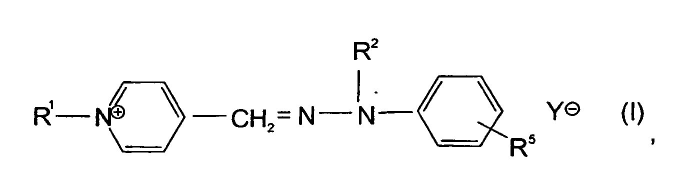 wherein R¹ and R² stand for hydrogen, a CH₃- or C₂H₅- group and R⁵ is hydrogen, -OCH₃ or -OC₂H₅ and Y⁻ is an anion,
b) at least one amphoteric and/or zwitterionic surfactant, and
c) at least one cationic surfactant.

2. Hair dyeing composition according to claim 1, comprising a dyestuff of the general formula (I), wherein R¹ and R² stand for a methyl group, R⁵ is hydrogen, and Y⁻ stands for a methosulfate group.

3. Hair dyeing composition according to claim 1, comprising a dyestuff of the general formula (I), wherein R¹ and R² each stand for a methyl group, R⁵ is a methoxy group and Y⁻ stands for Cl⁻.

4. Hair dyeing composition according to one or more of claims 1 to 3, containing coco amidobetaine.

## Revendications

1. Agent de coloration des cheveux, qui contient
a) au moins un colorant sélectionné parmi les composés du groupe 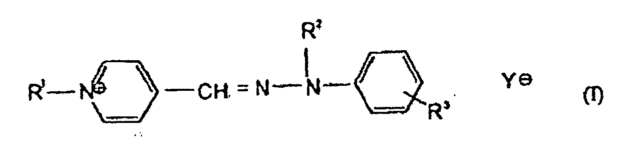 dans lequel R¹ et R² représentent l'hydrogène, un groupe CH₃ ou un groupe C₂H₅, R⁵ représente de l'hydrogène, -OCH₃ ou -OC₂H₅ et Y⁻ représente un anion,
b) au moins un agent tensioactif amphotère et/ou zwitterionique et
c) au moins un agent tensioactif cationique.

2. Agent de coloration des cheveux selon la revendication 1, qui contient un colorant de la formule générale (I) dans laquelle R¹ et R² représentent un groupe méthyle, R⁵ représente l'hydrogène et Y⁻ représente un groupe méthosulfate.

3. Agent de coloration des cheveux selon la revendication 1, qui contient un colorant de la formule générale (I) dans laquelle R¹ et R² représentent chacun un groupe méthyle, R⁵ représente un groupe méthoxy et Y⁻ représente Cl⁻.

4. Agent de coloration des cheveux selon l'une ou plusieurs des revendications 1 à 3, qui contient de la cocoamidobétaïne.
